# EUROPÄISCHE PATENTANMELDUNG

(11) **EP 2 016 892 A1**
(43) Veröffentlichungstag der Anmeldung: **21.01.2009**
(21) Anmeldenummer: 08158972.3
(22) Anmeldetag: 25.06.2008
(51) Int. Cl.: A61B 5/00, A61B 5/053

(54) **Implantierbares medizinisches Gerät**

(30) Priorität: 20.07.2007 DE 102007034042
(71) Anmelder: BIOTRONIK CRM Patent AG, 6341 Baar (CH)
(72) Erfinder: Lazarus, Arnaud, 92200 Neuilly sur Selne (FR); Albus, Marco, 12527 Berlin (DE); Bode, Sven, 12203 Berlin (DE); Elsner, Joachim, 14059 Berlin (DE); Kespohl, Stefanie, 10435 Berlin (DE)
(74) Vertreter: Lindner-Vogt, Karin L.

(57) **Zusammenfassung**

Die Erfindung betrifft ein implantierbares medizinisches Gerät, welches in einem Gehäuse Sensoren zum Erkennen des Gesundheitszustandes des das implantierbare medizinische Gerät umgebenden Körpergewebes aufweist.

## Beschreibung

Die Erfindung betrifft ein implantierbares medizinisches Gerät mit einem Gehäuse, welches eine Steuereinheit und eine mit der Steuereinheit verbundene Telemetrieeinheit für eine drahtlose Signalübertragung zu einem externen Gerät enthält.

Derartige medizinische Geräte sind beispielsweise Herzschrittmacher oder Defibrillatoren, die unter der Haut eines Patienten implantiert werden und dort für viele Jahre ihren Dienst versehen sollen.

Die Implantation des medizinischen Gerätes ist dabei mit einer Operation verbunden, die eine Wundheilung nach sich zieht. In den meisten Fällen verläuft die Wundheilung unproblematisch. Dennoch kommt es im postoperativen Verlauf nach Implantation eines implantierbaren medizinischen Gerätes immer wieder zu Komplikationen in der Wundheilung. In einer Studie wird von einer Komplikationsrate im Zusammenhang der Implantation von Herzschrittmachern in ca. 1 % der Fälle berichtet.

Um Komplikationen so schnell wie möglich zu erkennen, werden in der ersten Zeit nach der Implantation engmaschige Nachsorgeuntersuchungen durchgeführt. Dazu ist regelmäßig das Zusammenkommen von Patient und Arzt erforderlich. Üblicherweise wird der Patient zu solchen Nachsorgeuntersuchungen einbestellt. Im Falle häufiger Nachsorgeuntersuchungen ist der Aufwand erheblich. Sind die Nachsorgeuntersuchungen seltener, ist eine Früherkennung von Komplikationen nicht möglich.

Die Erfindung hat die Aufgabe, dieses Problem so weit wie möglich zu lösen. Erfindungsgemäß wird diese Aufgabe durch ein implantierbares medizinisches Gerät der eingangs genannten Art gelöst, das wenigstens einen mit der Steuereinheit verbundenen Sensor aufweist, der ausgebildet ist, eine für einen Gewebezustand charakteristische Gewebeeigenschaft von nach Implantation des implantierbaren medizinischen Gerätes in dessen Umgebung befindlichem Körpergewebe zu erfassen. Ein derartiges implantierbares medizinisches Gerät ermöglicht es, den Wundheilungsprozess nach Implantation des implantierbaren medizinischen Gerätes automatisch und fortlaufend durch das implantierbare medizinische Gerät selbst überwachen zu lassen und gegebenenfalls ein Warnsignal über die Telemetrieeinheit des implantierbaren medizinischen Gerätes zu senden und/oder einen Patientenalarm zu veranlassen.

Ein Warnsignal in diesem Sinne ist ein eindeutiges, auf eine Unregelmäßigkeit im Heilungsprozess hinweisendes Ereignis, das als Datenpaket übertragen werden kann oder aber auch als Patientenalarm direkt erfolgen kann.

Eine Telemetrieeinheit eines implantierbaren medizinischen Gerätes ermöglicht einen berührungslosen Übertragungsweg von Daten oder Datenpaketen aus diesem implantierbaren medizinischen Gerät. Dabei kann eine Verschlüsselung vorgesehen werden.

Unter einer mittelbaren Datenübertragung zu einem externen Server versteht man im Sinne der Erfindung das hochfrequente drahtlose Kommunizieren der Telemetrieeinheit des implantierbaren medizinischen Gerätes mit einer Schnittstelle eines externen Patientengeräts. Das Patientengerät wiederum stellt die Verbindung zu einem externen Server her.

Unter einer unmittelbaren Datenübertragung zu einem externen Server im erfindungsgemäßen Sinne versteht man die hochfrequente Kommunikation der Telemetrieeinheit des implantierbaren medizinischen Geräts direkt mit einer Schnittstelle eines externen Servers. Die Übertragung erfolgt mit einer Frequenz von 800, 900, 1800 MHz oder jeder anderen geeigneten Frequenz.

Ein Patientenalarm im Sinne der Erfindung ist ein Alarm, der nicht über die oben genannten Übertragungswege erfolgt, sondern eine direkte Rückmeldung an den Patienten liefert. Dieser Patientenalarm kann einerseits bei Vorhandensein auf dem externen Patientengerät oder aber bei Nicht-Vorhandensein eines externen Patientengerätes im implantierbaren medizinischen Gerät über akustische oder haptische Signale erfolgen. Dieser Alarm soll den Patienten anregen, proaktiv den Arzt aufzusuchen.

Weitere geeignete Warnsignale können erfindungsgemäß vorgesehen werden wie beispielsweise das Abrufen der Alarme bei einer Nachsorgeuntersuchung über eine Spule, die auf der Haut über dem implantierbaren medizinischen Gerät aufgelegt wird.

In diesem Zusammenhang ist die Steuereinheit des implantierbaren medizinischen Gerätes vorzugsweise so ausgebildet, dass sie von dem Sensor oder von den Sensoren stammende Sensorausgangssignale derart verarbeitet und auswertet, dass die Steuereinheit selbsttätig ein jeweiliges Sensorausgangssignal oder eine Mehrzahl zeitlich zusammenhängender Sensorausgangssignale einem gesunden Gewebezustand oder einem krankhaften, insbesondere einem entzündeten Gewebezustand zuordnet. Diese Zuordnung erfolgt im einfachsten Fall durch Vergleich eines jeweiligen Sensorausgangssignals mit einem entsprechenden Vergleichssignal. Dabei kann ein jeweiliges Sensorausgangssignal selbst oder dessen zeitliche Ableitung, beispielsweise in Form eines Differenzen-Quotienten, mit einem entsprechenden Vergleichswert verglichen werden.

In diesem Zusammenhang ist die Steuereinheit derart ausgebildet, dass sie durch Zuordnen des Sensorausgangssignals oder der Sensorausgangssignale zu einem entzündeten Gewebezustand einen entzündeten Gewebezustand detektiert und daraufhin gegebenenfalls ein Warnsignal über die Telemetrieeinheit (58) sendet und/oder einen geeigneten Patientenalarm veranlasst.

Besonders vorteilhaft ist es, wenn das implantierbare medizinische Gerät mehrere Sensoren aufweist, die Sensorausgangssignale liefern, welche einen Hinweis auf einen Gewebezustand liefern. Dann kann die Steuereinheit dazu ausgebildet sein, einen krankhaften oder gesunden Gewebezustand nicht nur an Hand eines einzigen Ausgangssignals zu detektieren, sondern an Hand mehrerer Ausgangssignale. In diesem Sinne können jeweilige Einzelwerte verschiedener Ausgangssignale zu einem Vektor zusammengefasst werden, die dann mit einem entsprechende Vergleichswerte enthaltenden Vektor zu vergleichen sind.

In diesem Zusammenhang kann die Steuereinheit dazu ausgebildet sein, ein Sensorausgangssignal oder mehrere Sensorausgangssignale mit einem oder mehreren Vergleichswerten zu vergleichen, die ein gesundes Körpergewebe charakterisieren und außerdem das Sensorausgangssignal oder die Sensorausgangssignale mit entsprechenden Vergleichswerten zu vergleichen, die krankhaftes Körpergewebe charakterisieren.

Wenn der erste Vergleich negativ ausfällt (kein gesundes Gewebe) und der zweite Vergleich positiv (krankhaftes Gewebe), dann ist dies ein deutlicher Hinweis darauf, dass das Körpergewebe tatsächlich krankhaft ist. Fällt hingegen der erste Vergleich positiv aus und der zweite Vergleich negativ, dann ist es ein deutlicher Hinweis darauf, dass das Körpergewebe gesund ist. Fallen beide Vergleiche jeweils positiv oder jeweils negativ aus, dann ist das ein Hinweis darauf, dass eine eindeutige Bestimmung des Körpergewebezustandes auf Grund der vorliegenden Sensorausgangssignale nicht möglich ist.

Um wie gewünscht Nachsorgeuntersuchungen möglichst einzusparen, ist es besonders vorteilhaft, wenn die Steuereinheit des implantierbaren medizinischen Gerätes dazu ausgebildet ist, nach Detektieren eines krankhaften oder entzündeten Gewebezustands in Folge der Auswertung des Sensorausgangssignals oder der Sensorausgangssignale gegebenenfalls ein Warnsignal über die Telemetrieeinheit des implantierbaren medizinischen Gerätes zu senden und/oder einen Patientenalarm zu veranlassen. Dieses Warnsignal kann einem betreuenden Arzt zugeleitet werden, der daraufhin Kontakt mit dem Patienten aufnehmen und entsprechende weitere Maßnahmen ergreifen kann.

In diesem Zusammenhang ist es ebenfalls vorteilhaft, wenn die Steuereinheit dazu ausgebildet ist, eine regelmäßige Übertragung der Sensorausgangssignale über die Telemetrieeinheit zu veranlassen und/oder einen Patientenalarm auszulösen, wenn die Steuereinheit für die automatische Erkennung eines möglichen krankhaften Gewebezustand an Hand der Konstellation der Sensorausgangssignale selbst nicht ausgebildet ist beziehungsweise diesen Gewebezustand anhand der oben genannten Vergleichswerte nicht bestimmen kann. Im ersten Falle ermöglicht dies einem betreuenden Arzt, die Sensorausgangssignale selbst zu inspizieren und einen möglichen krankhaften Gewebezustand an Hand einer Konstellation der Sensorausgangssignale zu erkennen. Außerdem kann sich der Arzt auf diese Weise regelmäßig vom Gesundheitszustand seines Patienten überzeugen. Im zweiten Falle ermöglicht dies dem Patienten, proaktiv einen Arzt aufzusuchen.

Ein geeigneter Sensor ist ein Temperatursensor, der so angeordnet und ausgebildet ist, dass er nach Implantation des implantierbaren medizinischen Gerätes eine Temperatur des, das Gehäuse umgebende, Körpergewebe erfasst. Solche Temperatursensoren sind als Thermoelemente oder Infrarotsensoren bekannt. Die Steuereinheit ist in diesem Zusammenhang dazu ausgebildet, ein von dem Temperatursensor als Sensorausgangssignal stammendes Temperatursignal mit einem Vergleichstemperaturwert zu vergleichen und bei Überschreiten des Vergleichstemperaturwertes einen krankhaften Gewebezustand zu detektieren. Insbesondere ist es vorteilhaft, wenn die Steuereinheit ausgebildet ist, einen krankhaften Gewebezustand daraus zu erkennen, dass das Temperatursignal für mehr als 12 Stunden ein einer Körpertemperatur von 37,0° bis 40,0° Celsius entsprechendes Temperaturvergleichssignal überschreitet. Der Temperatursensor kann in seiner Sensitivität manuell so eingestellt werden, dass der Schwellwert, der die Steuereinheit dazu veranlasst, gegebenenfalls ein Warnsignal über eine Telemetrieeinheit zu senden und/oder einen geeigneten Patientenalarm zu veranlassen, zwischen den genannten Temperaturwerten liegt.

Hintergrund ist, dass die Kerntemperatur im Bereich der Wunde weitgehend konstant bleiben soll. Eine lang andauernde Erhöhung der Temperatur von mehr als 0,5° Celsius über eine Grenze von 36,5° Celsius von mehr als 12 Stunden pro Tag ist ein geeigneter Indikator für eine entzündliche Reaktion des umliegenden Gewebes. Dabei ist zu berücksichtigen, dass es bei unmittelbarer subkutaner Lage des Implantats gelegentlich zu einer Erhöhung der Temperatur kommen kann, sodass die alleinige Temperaturerhöhung selbst (also unabhängig von deren Dauer) nicht als Indikator ausreicht. Beispielsweise kommt es auch dann zu einer Temperaturerhöhung, wenn sich ein Patient nach dem Schwimmen in die Sonne legt. Ein den Patienten versorgender Arzt muss bei einer dauerhaften Temperaturerhöhung um mehr als einen Grad prüfen, ob dieser auf eine Entzündung der Wunde oder beispielsweise auf ein Fieber mit anderer Ursache zurückgeht.

Ein ebenfalls geeigneter Sensor, der in Kombination mit dem Temperatursensor oder alternativ dazu eingesetzt werden kann, ist ein Gewebeflüssigkeitssensor. In diesem Falle ist die Steuereinheit ausgebildet, einen krankhaften Gewebezustand an Hand eines charakteristischen Anstiegs des Sensorausgangssignals des Gewebeflüssigkeitssensors zu detektieren und gegebenenfalls daraufhin ein Warnsignal über die Telemetrieeinheit zu senden und/oder einen geeigneten Patientenalarm zu veranlassen. Der Anstieg des Sensorausgangssignals charakterisiert dabei die steigende Menge von Gewebeflüssigkeit und/oder das Vorhandensein von Gewebeflüssigkeit in der Umgebung des implantierbaren medizinischen Geräts. Eine vorteilhafte Variante dieser Ausführungsform besteht darin, dass die Steuereinheit ausgebildet ist, einen Signalanstieg oder Abfall des Sensorausgangsignals des Gewebeflüssigkeitssensors durch Bilden eines repräsentativen Differenzen-Quotienten zu ermitteln und einen krankhaften Gewebezustand durch Vergleich dieses differenten Quotienten mit einem vorgegebenen, für einen charakteristischen Anstieg der Gewebeflüssigkeit typischen Vergleichswert, zu detektieren.

Ein geeigneter Gewebeflüssigkeitssensor ist beispielsweise ein Impedanzsensor, der zwei an dem Gehäuse des implantierbaren medizinischen Gerätes angeordnete Elektroden zur Stromeinspeisung und Spannungsmessung (oder Spannungseinspeisung und Strommessung) aufweist und ausgebildet ist, eine zwischen den Elektroden herrschende Impedanz zu messen und ein entsprechendes Sensorausgangssignal zu erzeugen. Dieses Sensorausgangssignal nimmt mit zunehmender Gewebeflüssigkeit typischerweise ab, sodass die Steuereinheit vorzugsweise dazu ausgebildet ist, einen Anstieg der Gewebeflüssigkeit an Hand eines charakteristischen Signalabfalls des Sensorausgangssignals des Impedanzsensors zu detektieren und gegebenenfalls daraufhin ein Warnsignal über die Telemetrieeinheit zu senden und/oder einen geeigneten Patientenalarm zu veranlassen.

Ein alternativer Sensor für die Gewebeflüssigkeit ist beispielsweise ein Ultraschalltransducer.

Ein weiterer, für den Einsatz im Rahmen der Erfindung, geeigneter Sensor ist ein pH-Wert-Sensor, der ausgebildet ist, ein repräsentierendes Sensorausgangssignal zu liefern, welches nach der Implantation des implantierbaren medizinischen Gerätes den pH-Wert der an dessen Gehäuse angrenzender Körperflüssigkeit darstellt. Dabei ist die Steuereinheit so ausgebildet, dass sie auf ein Sensorausgangssignal, das auf einen Abfall des pH-Wertes der Körperflüssigkeit in der Umgebung des Gehäuses hinweist, anspricht und einen krankhaften Gewebezustand detektiert.

Das implantierbare medizinische Gerät kann darüber hinaus auch einen Lagesensor als geeigneten Sensor aufweisen. Der Lagesensor ist ausgebildet, die Ausrichtung des implantierbaren medizinischen Gerätes relativ zur Richtung der Erdanziehung zu erfassen. Die Steuereinheit ist ausgebildet, Lagesensorausgangssignale über einen mehrere Tage dauernden Zeitraum zu erfassen und auszuwerten und auf eine langfristige, dauerhafte Veränderung der Lagesensorausgangssignale, die auf Veränderungen der relativen Position zwischen dem implantierbaren medizinischen Gerät und einem Patientenkörper hinweisen, anzusprechen und gegebenenfalls daraufhin ein Warnsignal über die Telemetrieeinheit des implantierbaren medizinischen Gerätes zu senden und/oder einen Patientenalarm zu veranlassen. Es ist nämlich möglich, dass das implantierbare medizinische Gerät durch krankhafte Veränderungen, bis hin zur Auflösung des umgebenden Gewebes seine Lage am Implantationsort verändert. Zudem kann durch externe Manipulation die Lage des implantierten Gerätes verändert werden. Eine dieser Manipulationen wird unter anderem "Twiddler-Syndrom" genannt, welches als "ineffektive Impulsgabe eines transvenös-intrakardialen Herzschrittmachers als Folge einer Elektrodendislokation, die nach multifaktoriell aufgetretener Rotation des Impulsgebers möglich ist" definiert wird (aus Roche-Lexikon Medizin, 4. Auflage). Die Rotation des Impulsgebers, der im Sinne dieser Erfindung ein implantierbares medizinisches Gerät ist, erfolgt beispielsweise durch den Patienten, in den dieser Impulsgeber implantiert wurde.

Durch die an einem implantierbaren medizinischen Gerät häufig angeschlossenen Elektroden (beispielsweise im Falle eines Herzschrittmachers) und den meist einseitigen Schwerpunkt ist dabei eine Parallelverschiebung des implantierbaren medizinischen Gerätes im Körper des Patienten sehr unwahrscheinlich. Vielmehr wird es wenigstens zusätzlich zu einer Rotationsbewegung des implantierbaren medizinischen Gerätes zur Flächennormale (also in Bezug auf die Richtung zum Erdgravitationszentrum) kommen. Die Lage eines implantierbaren medizinischen Gerätes zur Flächennormale (Ausrichtung zum Erdgravitationszentrum) lässt sich beispielsweise mit Sensoren auf Piezobasis erfassen. Um eine dauerhafte Lageänderung des implantierbaren medizinischen Gerätes relativ zum Körper des Patienten erkennen zu können, müssen die Lagesensorausgangssignale zunächst nach der Implantation kalibriert werden. Außerdem liefert ein derartiger Lagesensor von der Aktivität eines Patienten abhängige Signale und kann daher auch als Aktivitätssensor verwendet werden. Dadurch ist auch die eigentliche Änderung der Lage des implantierbaren medizinischen Gerätes gegenüber dem Körper des Patienten schwieriger zu ermitteln. Die Steuereinheit muss daher eine langfristige mittlere Lageänderung bestimmen und eine Änderung derselben detektieren. Dies kann beispielsweise dadurch geschehen, dass die Steuereinheit für diese Lagesensorausgangssignale Tageshistogramme erstellt und eine dauerhafte Verschiebung innerhalb dieser aufeinander folgenden Tageshistogramme erfasst. Mit Hilfe der Tageshistogramme lassen sich auch die verschiedenen Positionen eines Patienten (aufrecht, liegend etc.) bestimmen und ein Referenzsignal für die Lage des implantierbaren medizinischen Gerätes bei aufrecht stehendem Patienten bestimmen. Eine Abweichung von diesem Referenzsignal würde einen Hinweis auf eine Verschiebung des implantierbaren medizinischen Gerätes innerhalb des Patienten liefern.

Weiterhin, und zwar zusätzlich oder alternativ zu den bereits beschriebenen Sensoren, kann auch die Telemetrieeinheit in Verbindung mit der Steuereinheit selbst einen geeigneten Sensor darstellen. Dazu ist die Telemetrieeinheit mit ihrem Hochfrequenzsender und - empfänger in Kombination mit der Steuereinheit dazu ausgebildet, eine elektrische Permittivität der Umgebung des implantierbaren medizinischen Gerätes zu erfassen. Die elektrische Permittivität beschreibt das Absorptionsverhalten elektromagnetischer Wellen, wie sie vom Hochfrequenzsender der Telemetrieeinheit ausgesendet werden, in einem Medium sowie das Reflexionsverhalten an Grenzflächen. Eine Veränderung der elektrischen Permittivität über einen längeren Zeitraum deutet auf eine Veränderung des Körpergewebes hin. Durch einen im implantierbaren medizinischen Gerät integrierten Hochfrequenzsender und -empfänger kann die elektrische Permittivität, beispielsweise in bestimmten Zeitintervallen (z. B. täglich) gemessen werden. Der Verlauf der elektrischen Permittivität kann über einen längeren Zeitraum aufgezeichnet werden und als Diagnose für den Wundheilungsprozess verwendet werden.

Dementsprechend ist die Steuereinheit vorzugsweise dazu ausgebildet, die elektrische Permittivität in regelmäßigen Zeitabständen zu erfassen und eine Veränderung der elektrischen Permittivität als Zeichen einer Veränderung des Gewebezustandes zu detektieren und gegebenenfalls ein Warnsignal über die Telemetrieeinheit des implantierbaren medizinischen Gerätes zu senden und/oder einen Patientenalarm zu veranlassen. Eine geeignete Rate für das Messen der Permittivität ist ein bis fünf Mal täglich.

Wie zuvor schon angedeutet, ist es vorteilhaft, nicht nur ein einziges Sensorausgangssignal zum Detektieren eines gesunden oder eines krankhaften Körpergewebezustandes heranzuziehen, sondern eine Kombination von Sensorausgangssignalen zu betrachten. In diesem Zusammenhang ist es besonders vorteilhaft, wenn die Steuereinheit ausgebildet ist, einen gleichzeitigen Anstieg des Temperatursignals und einen charakteristischen Anstieg des die Gewebeflüssigkeit anzeigenden Sensorausgangssignals des Gewebeflüssigkeitssensors zu detektieren und gegebenenfalls ein Warnsignal über die Telemetrieeinheit des implantierbaren medizinischen Gerätes zu senden und/oder einen Patientenalarm zu veranlassen.

Die Steuereinheit kann weiterhin dazu ausgebildet sein, zusätzlich zur Körpergewebetemperatur und zur Gewebeflüssigkeit auch den pH-Wert des Körpergewebes auszuwerten und einen krankhaften Gewebezustand dann zu erkennen, wenn der Temperatursensor einen Anstieg der Körpertemperatur anzeigt, der Gewebeflüssigkeitssensor einen Anstieg der Gewebeflüssigkeit anzeigt und der pH-Wert-Sensor einen Abfall des pH-Wertes anzeigt.

Neben dem Aussenden eines Warnsignals im Falle der Detektion eines potentiell krankhaften Gewebezustandes kann das implantierbare medizinische Gerät auch dazu ausgebildet sein, ein entzündungshemmendes Medikament an den Körper des Patienten auszugeben. Daher ist ein implantierbares medizinisches Gerät bevorzugt, das ein steuerbares Medikamentendepot aufweist, welches mit der Steuereinheit verbunden und ausgebildet ist, auf ein Steuersignal der Steuereinheit hin ein Medikament an die Umgebung des implantierbaren medizinischen Gerätes abzugeben. Die Steuereinheit ist dabei dazu ausgebildet, auf das Detektieren eines potentiell krankhaften Gewebezustandes hin ein Steuersignal an das Medikamentendepot abzugeben, so dass eine Medikamentenabgabe durch das Depot erfolgt. Vorzugsweise ist das Medikamentendepot mit einem entzündungshemmenden Medikament, beispielsweise mit einem Medikament aus der Gruppe der Steroide wie beispielsweise Dexamethason und/oder Prednisolon gefüllt. Weitere vorteilhafte Ausgestaltungen der Erfindung ergeben sich aus der nachfolgenden Beschreibung eines Ausführungsbeispiels. Die Beschreibung erfolgt mit Bezug auf die Figuren. Diese zeigen:
- Figur 1:: einen Überblick über ein Patientenüberwachungssystem für einen Patien- ten mit einem implantierbaren medizinischen Gerät;
- Figur 2:: ein implantierbares medizinisches Gerät in Form eines Herzschrittmachers in Kombination mit einem externen Patientengerät und einem Herzen eines Menschen;
- Figur 3:: ein Blockschaltbild eines erfindungsgemäßen implantierbaren medizini- schen Gerätes.

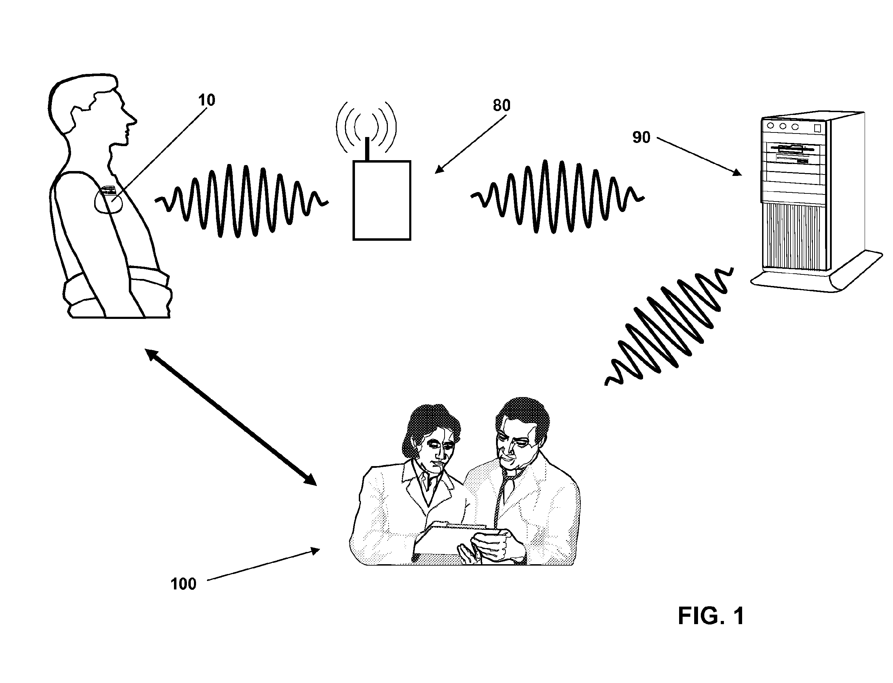

Das beispielhafte System zur Patientenüberwachung in Figur 1 zeigt einen Patienten mit einem implantierbaren medizinischen Gerät 10 in Form eines Herzschrittmachers. Dieser weist eine Telemetrieeinheit auf, um drahtlos mit einem externen, sich in der Regel in der Nähe des Patienten befindenden Gerät 80 zu kommunizieren. Das externe Gerät 80 wiederum kann über eine zweite Schnittstelle drahtlos oder drahtgebunden, beispielsweise über Telefonverbindungen, mit einem zentralen Server 90 in einem zentralen Service-Center kommunizieren. Über das zentrale Service-Center ist es wiederum möglich, einen Arzt oder ein Ärzteteam 100 zu informieren, falls beispielsweise das implantierbare medizinische Gerät einen kritischen Zustand detektiert und über das externe Gerät 80 einen zentralen Server 90 signalisiert. Das Ärzteteam 100 kann dann direkten Kontakt mit dem Patienten aufnehmen.

Ein typisches implantierbares medizinisches Gerät ist ein Herzschrittmacher 10, wie er in Figur 1 abgebildet ist. Dieser ist unter der Haut des Patienten implantiert und befindet sich in einer sogenannten Herzschrittmachertasche. Nach der Implantation des Herzschrittmachers muss das die Herzschrittmachertasche bildende Gewebe ausheilen. Dabei kann es zu einer lokalen ödematösen Wasseransammlung im Rahmen einer entzündlichen Reaktion in der Herzschrittmachertasche kommen. In fortgeschrittenen Stadien der Entzündungsreaktion kann es aber auch zu anderen krankhaften Gewebeveränderungen, wie beispielsweise Vereitern der Tasche, und im schlimmsten Falle zu einer Nekrotisierung bzw. Auflösung des Gewebes der Herzschrittmachertasche kommen, so dass das Implantat seine ursprüngliche Lage verändert. Aus diesem Grund ist es wichtig, entzündliche Reaktionen möglichst frühzeitig zu erkennen. Die Untersuchung auf diese entzündlichen Reaktionen wird heutzutage üblicherweise im Rahmen von direkten Nochsorgeuntersuchungen eines Arztes durchgeführt.

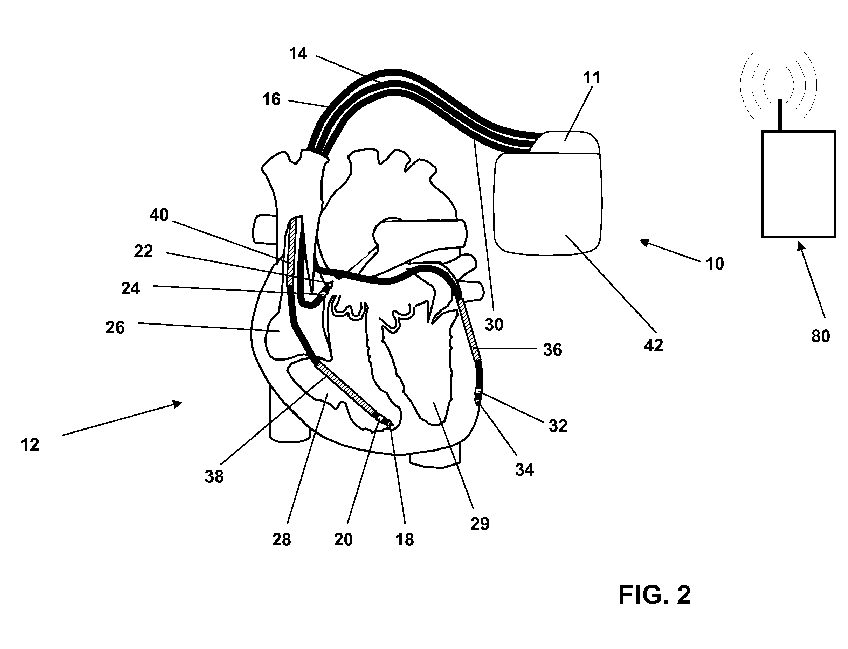

Figur 2 zeigt noch einmal den Herzschrittmacher 10 von außen, sowie typische Elektrodenleitungen 14, 16 und 30, über die der Herzschrittmacher 10 mit Elektroden 18, 20, 22, 24, 32, 34, 36, 38 und 40 mit dem Herzen 12 eines Patienten verbunden ist und mindestens eine der vier Kammern des Herzens 12 wie beispielsweise das rechte Atrium 26 oder den rechten Ventrikel 28, welche für die Sauerstoffversorgung eines Patienten besonders wichtig sind, über diese Elektroden 18, 20, 22, 24, 32, 34, 36, 38, und 40 stimulieren kann.

Der Herzschrittmacher 10 besitzt ein Gehäuse 40, üblicherweise aus einem biokompatiblen Werkstoff, wie beispielsweise Titan, das elektronische Komponenten einschließt. An dem Gehäuse 42 ist ein sogenannter Header 11 angebracht, der Kontaktbuchsen zum Anschließen der Elektrodenleitungen 14, 16 und 30 aufweist. Der Header besteht häufig aus transparentem Kunststoff.

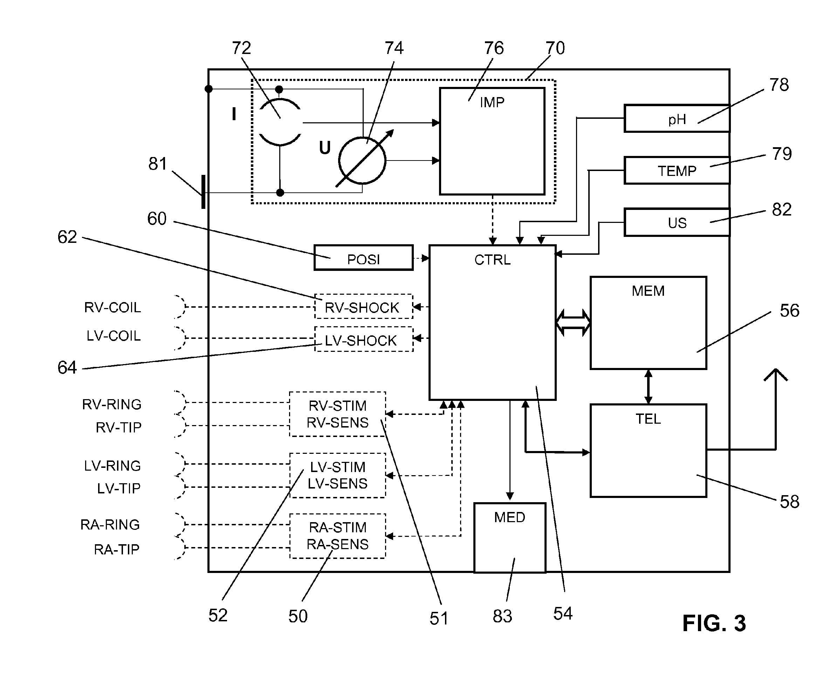

Figur 3 ist ein Blockschaltbild des Herzschrittmachers 10 aus Figur 2. Anzumerken ist, dass der Herzschrittmacher 10 nicht nur ein reiner Herzschrittmacher ist, sondern gleichzeitig auch ein Defibrillator, der über Schockelektroden 36, 38 und 40 Defibrillationsschocks an das Herz abgeben kann. Außerdem ist der Herzschrittmacher 10 zur Stimulation sowohl des rechten Ventrikels 28 als auch des linken Ventrikels 29 des Herzens 12 ausgebildet. Hierzu weist der Herzschrittmacher 10 die in Figur 3 gestrichelt dargestellten Komponenten, nämlich eine rechtsatriale Stimulationseinheit (ra-stim) und eine atriale Sensingeinheit (ra-sens), gemeinsam mit dem Bezugszeichen 50 bezeichnet, auf sowie eine rechtsventrikuläre Stimulationseinheit (rv-stim) und eine rechtsventrikuläre Sensingeinheit rv-sens (gemeinsam mit dem Bezugszeichen 51 bezeichnet) sowie eine linksventrikuläre Stimulationseinheit (Iv-stim) und eine linksventrikuläre Sensingeinheit (Ivsens) (gemeinsam mit dem Bezugszeichen 52 versehen) auf. Diese Einheiten erlauben das Stimulieren des rechten Atriums des rechten Ventrikels und des linken Ventrikels des Herzens im sogenannten Demand-Modus, in dem eine Abgabe von Stimulationsimpulsen unterbleibt, wenn innerhalb eines Escape-Intervalls durch die entsprechende SensingEinheit eine natürliche Kontraktion der jeweiligen Herzkammer erfasst wird. Außerdem ist eine rechtsventrikuläre Schockeinheit 62 und eine linksventrikuläre Schockeinheit 64 vorgesehen, die jeweils mit entsprechenden Anschlüssen für den Anschluss der entsprechenden Elektrodenleitungen verbunden sind.

Im Zusammenhang mit der vorliegenden Erfindung interessant sind die nicht gestrichelt dargestellten Komponenten, nämlich allen voran eine zentrale Steuereinheit CTRL 54, die zum Einen mit einer Speichereinheit MEM 56 und zum Anderen mit einer Telemetrieeinheit TEL 58 verbunden ist. Darüber hinaus ist die Steuereinheit CTRL 54 mit einer Reihe von Sensoren verbunden, mit denen sich Parameter bestimmen lassen, die einen Hinweis auf den Gesundheitszustand des den Herzschrittmacher 10 umgebenden Körpergewebes geben.

Ein Sensor ist ein Positionssensor 60, der beispielsweise mit Hilfe von piezoelektrischen Elementen in der Lage ist, die Richtung der Schwerkraft in Bezug auf den Herzschrittmacher 10 zu ermitteln. Gleichzeitig kann der Positionssensor 60 auch Lageänderungen und Beschleunigungen des Herzschrittmachers 10 erfassen und somit auch für die Aktivität des Patienten charakteristische Sensorausgangssignale liefern. Diese Sensorausgangssignale werden der Steuereinheit CTRL 54 zugeführt und in der eingangs beschriebenen Weise verarbeitet. Ein weiterer Sensor ist ein Impedanzsensor 70, der eine Konstantstromquelle 72 aufweist, sowie eine Spannungsmesseinheit 74, die beide mit einer Impedanzmesseinheit 76 verbunden sind. Die Konstantstromquelle 72 erzeugt paarweise Konstantstromimpulse mit sich abwechselnder Polung und gibt diese über zwei Elektroden ab, von denen eine von dem Gehäuse 42 des Herzschrittmachers selbst gebildet ist und die zweite Elektrode 80 isoliert davon auf dem Gehäuse 42 des Herzschrittmachers 10 angeordnet ist. Die Impedanzmesseinheit 76 ist mit der Steuereinheit 54 verbunden und liefert an diese ein Sensorausgangssignal, welches die Impedanz des den Herzschrittmacher 10 umgebenden Gewebes widerspiegelt. Diese Impedanz ist umso niedriger, je größer der Gewebeflüssigkeitsanteil in dem den Herzschrittmacher 10 umgebenden Körpergewebe ist. Die Auswertung dieses Impedanzsensorausgangssignals durch die Steuereinheit 54 erfolgt auf die eingangs beschriebene Weise.

Alternativ oder zusätzlich kann die Gewebeflüssigkeit des den Herzschrittmacher 10 umgebenden Körpergewebes auch mit einem Ultraschallsensor 82 erfasst werden, der hierzu einen Ultraschalltransducer aufweist - beispielsweise in Form eines piezoelektrischen Elementes -, der Ultraschallsignale aussenden und empfangen kann. Mit einem Gewebeflüssigkeitssensor in Form des Impedanzsensors 70 oder des Ultraschallsensors 82 kann ein Sensorausgangssignal gewonnen werden, welches vom Gewebeflüssigkeitsanteil des den Herzschrittmacher 10 umgebenen Körpergewebes abhängt. Im Normalfall befindet sich keine Flüssigkeit in der den Herzschrittmacher 10 umgebenden abheilenden Wunde. Allenfalls ist in den ersten 7 Tagen noch Flüssigkeit in der Wunde zu finden. Hier ist jedoch bei normaler Wundheilung von einer stetigen Abnahme der freien Flüssigkeit auszugehen. Daher ist ein Neuauftreten von freier Flüssigkeit bzw. eine Zunahme von Gewebeflüssigkeit nach Ablauf von 7 Tagen nach Implantation des Herzschrittmachers 10 ein Indikator für eine Störung im Wundheilungsprozess. Diese kann durch Auswerten des jeweiligen Sensorausgangssignals des Impedanzsensors 70 oder des Ultraschallsensors 82 auf die eingangs beschriebene Weise detektiert werden.

Ein weiterer Sensor ist ein pH-Wert-Sensor 78 mit dem sich der pH-Wert des den Herzschrittmacher 10 umgebenden Körpergewebes bestimmen lässt. Dieser pH-Wert liegt bei normalem Gewebe zwischen 7,0 und 8,0. Durch Anreicherung von sauren Valenzen durch eine Gewebehypoxie und Freisetzung von sauren Enzymen im Entzündungsgewebe verschiebt sich der pH-Wert in einen zunehmend sauren Bereich von 6 bis 7. Eine derartige Verschiebung des pH-Wertes und damit eine derartige Veränderung des Sensorausgangssignals des pH-Wert-Sensors 78 kann ebenfalls von der Steuereinheit 54 detektiert werden und als Anzeichen für eine Entzündung des Körpergewebes gewertet werden.

Schließlich kann - wie eingangs schon beschrieben - die Telemetrieeinheit 58 selbst zur Gewinnung eines den Gesundheitszustand des den Herzschrittmacher 10 umgebenden Körpergewebes charakterisierenden Ausgangssignals herangezogen werden, indem mit Hilfe des in der Telemetrieeinheit 58 vorgesehenen Hochfrequenzsenders und - empfängers die Permittivität des den Herzschrittmacher 10 umgebenden Körpergewebes bestimmt wird. Dies ist eingangs bereits beschrieben.

Ein besonders wichtiger Sensor ist ein Temperatursensor 79, der die Temperatur des den Herzschrittmacher 10 umgebenden Körpergewebes erfasst. Diese Temperatur liegt beim gesunden Menschen in der Größenordnung von 36,5°C. Steigt diese Temperatur dauerhaft um mehr als 1°C, also über 37,5°C an, ist dies ein Anzeichen für einen Entzündungsprozess, sofern der Patient nicht beispielsweise Fieber hat. Daher kann auch das Sensorausgangssignal des Temperatursensors 79 auf die eingangs beschriebene Weise zur Detektion eines entzündlichen Gewebezustandes herangezogen werden.

Wie eingangs ebenfalls beschrieben, ist die Kontrolleinheit 54 dazu ausgebildet, zur Bestimmung des Gesundheitszustandes des Körpergewebes nicht nur ein einziges Sensorausgangssignal auszuwerten, sondern mehrere Sensorausgangssignale in Kombination miteinander auszuwerten. Dabei erkennt die Steuereinheit 54 entzündetes Körpergewebe daran, dass das Sensorausgangssignal des Temperatursensors 79 eine für mehrere Stunden um wenigstens 1°C gestiegene Körpertemperatur anzeigt, das Sensorausgangssignal des Gewebeflüssigkeitssensors einen gesteigerten Gewebeflüssigkeitsanteil des den Herzschrittmacher 10 umgebenden Körpergewebes anzeigt und das Sensorausgangssignal des pH-Wert-Sensors einen pH-Wert unterhalb von 7 anzeigt.

Falls die Steuereinheit 54 eine derartige Parameterkonstellation detektiert und damit einen potentiell krankhaften Körpergewebezustand detektiert, löst sie das Versenden eines Warnsignals über die Telemetrieeinheit 58 aus. Dieses wird letztendlich über das externe Gerät 80 dem zentralen Server 90 zugestellt. Der zentrale Server 90 wiederum kann eine Warnung an einen jeweiligen, den Patienten betreuenden Arzt weiterleiten, beispielsweise per sms oder e-mail.

Zusätzlich weist der Herzschrittmacher 10 ein Medikamentendepot 83 auf, in dem sich ein entzündungshemmendes Medikamament aus der Gruppe der Steroide wie beispielsweise Dexamethason und/oder Prednisolon befindet. Das Medikamentendepot 83 ist so mit der Steuereinheit 54 verbunden, dass es infolge eines entsprechenden Steuersignals der Steuereinheit 54 das Medikament an die Umgebung des Herzschrittmachers 10 abgibt. Die Steuereinheit 54 gibt dieses Steuersignal dann aus, wenn sie, wie zuvor beschrieben, einen krankhaften Gewebezustand detektiert hat.

## Patentansprüche

1. Implantierbares medizinisches Gerät (10) mit einem Gehäuse (42), welches eine Steuereinheit (54) und eine mit der Steuereinheit (54) verbundene Telemetrieeinheit (58) für die drahtlose Signalübertragung zu einem externen Gerät enthält, **dadurch gekennzeichnet, dass** das implantierbare medizinische Gerät (10) wenigstens einen mit der Steuereinheit (54) verbundenen Sensor (60; 70; 78; 79; 82) aufweist, der ausgebildet ist, eine für einen Gewebezustand charakteristische Gewebeeigenschaft von nach Implantation des implantierbaren medizinischen Gerätes das Gehäuse umgebenden Körpergewebe zu erfassen.

2. Implantierbares medizinisches Gerät nach Anspruch 1, **dadurch gekennzeichnet, dass** die Steuereinheit (54) ausgebildet ist, von dem Sensor oder von den Sensoren stammende Sensorausgangssignale derart zu verarbeiten und auszuwerten, dass die Steuereinheit selbsttätig ein jeweiliges Sensorausgangssignal oder eine Mehrzahl zeitlich zusammenhängender Sensorausgangssignale einem gesunden Gewebezustand oder einem krankhaften, insbesondere einem entzündeten Gewebezustand zuordnet und/oder
dass die Steuereinheit (54) ausgebildet ist, durch Zuordnen des Sensorausgangssignals oder der Sensorausgangssignale zu einem entzündeten Gewebezustand einen entzündeten Gewebezustand zu detektieren und daraufhin gegebenenfalls ein Warnsignal über die Telemetrieeinheit (58) zu senden und/oder einen geeigneten Patientenalarm zu veranlassen und/oder
dass die Steuereinheit (54) ausgebildet ist, mehrere Sensorausgangssignale in Kombination miteinander auszuwerten, um einen krankhaften Gewebezustand zu detektieren und/oder
dass die Steuereinheit (54) ausgebildet ist, eine regelmäßige Übertragung der Sensorausgangssignale über die Telemetrieeinheit (58) zu veranlassen und/oder einen geeigneten Patientenalarm auszulösen.

3. Implantierbares medizinisches Gerät nach Anspruch 2, **dadurch gekennzeichnet, dass** die Steuereinheit (54) ausgebildet ist, ein Zuordnen des Sensorausgangssignals oder der Sensorausgangssignale zu einem gesunden Gewebezustand oder einem krankhaften Gewebezustand durch Vergleich des Sensorausgangssignals oder der Sensorausgangssignale mit charakteristischen Vergleichswerten durchzuführen.

4. Implantierbares medizinisches Gerät nach einem der Ansprüche 1 bis 3, **dadurch gekennzeichnet,**
**dass** der Sensor ein Temperatursensor (79) ist, der angeordnet und ausgebildet ist, nach Implantation des implantierbaren medizinischen Gerätes (10) eine Temperatur des, das Gehäuse (42) umgebende, Körpergewebes zu erfassen und dass die Steuereinheit (54) ausgebildet ist, ein von dem Temperatursensor (79) als Sensorausgangssignal stammendes Temperatursignal mit einem Vergleichtemperaturwert zu vergleichen und bei Überschreiten des Vergleichstemperaturwertes einen krankhaften Gewebezustand zu detektieren.

5. Implantierbares medizinisches Gerät nach Anspruch 4, **dadurch gekennzeichnet, dass** die Steuereinheit (54) ausgebildet ist, einen krankhaften Gewebezustand zu detektieren und gegebenenfalls daraufhin ein Warnsignal über die Telemetrieeinheit (58) zu senden und/oder einen geeigneten Patientenalarm zu veranlassen, wenn das Temperatursignal für mehr als 12 Stunden ein Temperaturvergleichssignal überschreitet, das einer vorherbestimmten Temperatur, welche vorzugsweise zwischen 37,0°C und 40,0°C liegt, entspricht.

6. Implantierbares medizinisches Gerät nach einem der Ansprüche 1 bis 6, **dadurch gekennzeichnet,**
**dass** der Sensor ein Gewebeflüssigkeitssensor (70; 82) ist und die Steuereinheit (54) ausgebildet ist, einen krankhaften Gewebezustand zu detektieren und daraufhin gegebenenfalls ein Warnsignal über die Telemetrieeinheit (58) zu senden und/oder einen geeigneten Patientenalarm zu veranlassen, wenn das Sensorausgangssignal des Gewebeflüssigkeitssensors einen charakteristischen Anstieg der Gewebeflüssigkeit anzeigt.

7. Implantierbares medizinisches Gerät nach Anspruch 6, **dadurch gekennzeichnet, dass** die Steuereinheit (54) ausgebildet ist, einen einen Signalanstieg oder -abfall repräsentierenden, Differenzenquotienten des Sensorausgangssignals des Gewebeflüssigkeitssensors zu bilden und durch Vergleich des Differenzenquotienten mit einem vorgegebenen Vergleichswert einen charakteristischen Anstieg der Gewebeflüssigkeit zu detektieren und/oder
dass der Gewebeflüssigkeitssensor ein Impedanzsensor (70) ist, der zwei an dem Gehäuse des implantierbaren medizinischen Gerätes angeordnete Elektroden (42, 81) zur Stromeinspeisung und Spannungsmessung aufweist und der ausgebildet ist, eine zwischen den Elektroden herrschende Impedanz zu messen und ein entsprechendes Sensorausgangssignal zu erzeugen und/oder
dass der Gewebeflüssigkeitssensor einen Ultraschalltransducer (82) aufweist.

8. Implantierbares medizinisches Gerät nach Anspruch 7, **dadurch gekennzeichnet, dass** die Steuereinheit (54) ausgebildet ist, einen charakteristischen Anstieg der Gewebeflüssigkeit anhand eines charakteristischen Signalabfalls des Sensorausgangssignals des Impedanzsensors (70) zu detektieren.

9. Implantierbares medizinisches Gerät nach einem der Ansprüche 1 bis 8, **dadurch gekennzeichnet,**
**dass** der Sensor ein pH-Wert-Sensor (78) ist, der ausgebildet ist, ein einen pH-Wert von nach Implantation des implantierbaren medizinischen Gerätes an dessen Gehäuse angrenzender Körperflüssigkeit repräsentierendes, Sensorausgangssignal zu liefern, und dass die Steuereinheit ausgebildet ist, auf einen Abfall des pH-Wertes der Körperflüssigkeit in der Umgebung des Gehäuses des implantierbaren medizinischen Gerätes anzusprechen und einen krankhaften Gewebezustand zu detektieren und daraufhin gegebenenfalls ein Warnsignal über die Telemetrieeinheit (58) zu senden und/oder einen geeigneten Patientenalarm zu veranlassen.

10. Implantierbares medizinisches Gerät nach einem der Ansprüche 1 bis 6, **dadurch gekennzeichnet, dass** das implantierbare medizinische Gerät (10) einen Lagesensor (60) aufweist, der ausgebildet ist, die Ausrichtung des implantierbaren medizinischen Gerätes (10) relativ zur Richtung der Schwerkraft zu erfassen und dass die Steuereinheit (54) ausgebildet ist, Lagesensorausgangssignale über einen mehrere Tage dauernden Zeitraum zu erfassen und auszuwerten und auf eine langfristige, dauerhafte Veränderung der Lagesensorausgangssignale, die auf eine Veränderung der relativen Position zwischen dem implantierbaren medizinischen Gerät (10) und einem Patientenkörper, in dem das implantierbare medizinische Gerät (10) implantiert ist, hinweisen, anzusprechen und daraufhin gegebenenfalls ein Warnsignal über die Telemetrieeinheit (58) zu senden und/oder einen geeigneten Patientenalarm zu veranlassen und/oder
dass die Steuereinheit (54) ausgebildet ist, ein jeweiliges Tageshistogramm der Lagesensorausgangssignale zu erstellen und eine dauerhafte Verschiebung in der Form aufeinander folgender Tageshistogramme zu detektieren.

11. Implantierbares medizinisches Gerät nach einem der Ansprüche 1 bis 3, **dadurch gekennzeichnet, dass** die Telemetrieeinheit (58) einen Hochfrequenzsender und - empfänger aufweist und in Kombination mit der Steuereinheit (54) dazu ausgebildet ist, eine elektrische Permittivität der Umgebung des implantierbaren medizinischen Gerätes (10) zu erfassen und/oder
dass die Steuereinheit (54) ausgebildet ist, die elektrische Permittivität in regelmäßigen Zeitabständen zu erfassen und eine Veränderung der elektrischen Permittivität als Zeichen einer Veränderung des Gewebezustandes zu detektieren und daraufhin gegebenenfalls ein Warnsignal über die Telemetrieeinheit (58) zu senden und/oder einen geeigneten Patientenalarm zu veranlassen und/oder
dass die Steuereinheit (54) ausgebildet ist, die elektrische Permittivität ein- bis fünfmal täglich zu erfassen.

12. Implantierbares medizinisches Gerät nach Anspruch 2, 4 und 6, **dadurch gekennzeichnet,**
**dass** die Steuereinheit (54) ausgebildet ist, einen gleichzeitigen Anstieg des Temperatursignals und ein einen charakteristischen Anstieg der Gewebeflüssigkeit anzeigendes Sensorausgangssignal des Gewebeflüssigkeitssensors zu detektieren und daraufhin gegebenenfalls ein Warnsignal über die Telemetrieeinheit (58) zu senden und/oder einen geeigneten Patientenalarm zu veranlassen.

13. Implantierbares medizinisches Gerät nach Anspruch 2, 4, 6 und 9, **dadurch gekennzeichnet,**
**dass** die Steuereinheit (54) ausgebildet ist, einen gleichzeitigen Anstieg des Temperatursignals, ein einen charakteristischen Anstieg der Gewebeflüssigkeit anzeigendes Sensorausgangssignal des Gewebeflüssigkeitssensors (70; 82) und ein einen Abfall des pH-Wertes kennzeichnendes Sensorausgangssignal des pH-Wert-Sensors (78) zu detektieren und daraufhin gegebenenfalls ein Warnsignal über die Telemetrieeinheit (58) zu senden und/oder einen geeigneten Patientenalarm zu veranlassen.

14. Implantierbares medizinisches Gerät nach einem der Ansprüche 2 bis 13, **dadurch gekennzeichnet, dass** das implantierbare medizinische Gerät (10) ein steuerbares Medikamentendepot (83) aufweist, welches mit der Steuereinheit (54) verbunden und ausgebildet ist, auf ein Steuersignal der Steuereinheit (54) hin ein Medikament an die Umgebung des implantierbaren medizinischen Gerätes (10) abzugeben and dass die Steuereinheit (54) ausgebildet ist, auf das Detektieren eines krankhaften Gewebezustands hin ein Steuersignal an das Medikamentendepot abzugeben, das eine Medikamentenabgabe durch das Medikamentendepot (83) bewirkt und/oder
dass das Medikamentendepot (83) ein entzündungshemmendes Medikament enthält.

15. Implantierbares medizinisches Gerät nach Anspruch 14, **dadurch gekennzeichnet, dass** das ein Medikament aus der Gruppe der Steroide, vorzugsweise Dexamethason und/oder Prednisolon enthalten ist.
